# EUROPEAN PATENT SPECIFICATION

(11) **EP 0 621 773 B1**
(45) Date of publication and mention of the grant of the patent: **25.11.1998**
(21) Application number: 93903070.6
(22) Date of filing: 13.01.1993
(51) Int. Cl.: A61K 9/127, A61K 31/20

(54) **FORMULATION AND USE OF CAROTENOIDS IN TREATMENT OF CANCER**
FORMULIERUNG UND VERWENDUNG VON CAROTENOIDEN IN DER KREBSBEHANDLUNG
FORMULATION A BASE DE CAROTENOIDES ET UTILISATION DE CES DERNIERS DANS LE TRAITEMENT DU CANCER

(30) Priority: 16.01.1992 US 822055
(43) Date of publication of application: 02.11.1994
(73) Proprietor: BOARD OF REGENTS THE UNIVERSITY OF TEXAS SYSTEM, Austin, Texas 78701 (US); Aronex Pharmaceuticals, Inc., The Woodlands, TX 77381-1191 (US)
(72) Inventor: MEHTA, Kapil, Houston, TX (US); PEREZ-SOLER, Roman, Houston, TX (US); LOPEZ-BERESTEIN, Gabriel, Houston, TX 77096 (US); LENK, Robert P., New Waverly, TX (US); HAYMAN, Alan C., The Woodlands, TX (US)
(74) Representative: Dost, Wolfgang, Dr.rer.nat., Dipl.-Chem.
(86) International application number: US9300233
(87) International publication number: WO9313751

(56) References cited:
- EP-A- 0 287 198
- WO-A-89/06977
- WO-A-89/11850
- WO-A-91/12794
- FR-A- 2 455 458

## Description

The present invention relates to therapeutic compositions of carotenoids encapsulated in liposomes or other lipid carrier particles.

It has been known for more than 50 years that retinoids, the family of molecules comprising both the natural and synthetic analogues of retinol (vitamin A), are potent agents for control of both cellular differentiation and cellular proliferation (Wolbach et al., J. Exp. Med., 42:753-777, 1925). Several studies have shown that retinoids can suppress the process of carcinogenesis in vivo in experimental animals (for reviews, see e.g., Bollag, Cancer Chemother. Pharmacol., 3:207-215, 1979, and Sporn et al., In Zedeck et al. (eds.), Inhibition of Tumor induction and development, pp. 71-100. New York: Plenum Publishing Corp., 1981). These results are now the basis of current attempts to use retinoids for cancer prevention in humans. Furthermore, there is extensive evidence which suggests that retinoids can suppress the development of malignant phenotype in vitro (for review, see e.g., Bertram et al., In: M.S. Arnott et al., (eds.), Molecular interactions of nutrition and cancer, pp 315-335. New York, Raven Press, 1982; Lotan et al., The modulation and mediation of cancer by vitamins, pp 211-223. Basel: S. Karger AG, 1983) thus suggesting a potential use of retinoids in cancer prevention. Also, recently it has been shown that retinoids can exert effects on certain fully transformed, invasive, neoplastic cells leading in certain instances to a suppression of proliferation (Lotan, Biochim. Biophys. Acta, 605:33-91, 1980) and in other instances to terminal differentiation of these cells, resulting in a more benign, non-neoplastic phenotype (see e.g., Brietman et al., Proc. Natl. Acad. Sci. U.S.A., 77:2936-2940, 1980).

Retinoids have also been shown to be effective in the treatment of cystic acne (see e.g., Peck, et al., New Engl. J. Med., 300:329-333, 1979). In addition to cystic acne, retinoid therapy has been shown to be effective in gram-negative folliculitis, acne fulminans, acne conglobata, hidradenitis suppuritiva, dissecting cellulitis of the scalp, and acne rosacea (see e.g., Plewig et al., J. Am. Acad. Dermatol., 6:766-785, 1982).

However, due to highly toxic side effects of naturally occurring forms of vitamin A (hypervitaminosis A) at therapeutic dose level, clinical use of retinoids has been limited (Kamm et al., In: The Retinoids. Sporn et al., (eds.), Academic Press, N.Y., pp 228-326, 1984; Lippman et al., Cancer Treatment Reports, 71:493-515, 1987). In free form, the retinoids may have access to the surrounding normal tissues which might be the basis of their profound toxicity to liver, central nervous system, and skeletal tissue.

Therefore, one potential method to reduce the toxicity associated with retinoid administration would be the use of a drug delivery system. The liposomal format is a useful one for controlling the topography of drug distribution in vivo. This, in essence, involves attaining a high concentration and/or long duration of drug action at a target (e.g. a tumor) site where beneficial effects may occur, while maintaining a low concentration and/or reduced duration at other sites where adverse side effects may occur (Juliano, et al., In: Drug Delivery Systems, Juliano ed., Oxford Press, N.Y., pp 189-230, 1980). Liposome-encapsulation of drug may be expected to impact upon all the problems of controlled drug delivery since encapsulation radically alters the pharmacokinetics, distribution and metabolism of drugs.

There are additional difficulties in using a liposomal formulation of a retinoid for therapeutic purposes. For example, it is often desirable to store the composition in the form of a preliposomal powder, but many prior formulations are not satisfactory for such use, because they either contain an inadequate amount of retinoid, or they generate undesirable liposomes when they are reconstituted in aqueous solution.

For compositions that are to be administered intravenously, typically the composition must provide at least about 100 mg of the active ingredient in a single container; if it contains a lesser amount of the active ingredient, an impractically large number of vials will be needed for dosing a single patient.

Typically a vial having a volume of 120ml(cc) is the largest that can be accommodated in a commercial freeze drier, and 50ml(cc) is the maximum volume of liquid that can be filled in such a vial. If more than 1 g of lipids are included in 50ml(cc) of liquid volume, the resulting liposomes after reconstitution have a size distribution which is not acceptable for parenteral administration. This is because the packing of the lipids during lyophilization is affected by the concentration of the lipids in the solution. Thus, the concentration of lipids in the solution must be limited. However, when this is done in previously-known liposomal retinoid formulations, the retinoid tends to crystallize, and separate from the liposomes shortly after reconstitution.

In order to both limit the concentration of lipids and supply a sufficient amount of retinoid, it is necessary to provide a molar ratio of retinoid to lipid greater than about 1 to 10. Previously known formulations have not had, and are believed not to be capable of having such a high packing of retinoid in the liposomes. Therefore, a need exists for improved compositions and methods which will minimize or eliminate the problems of the prior art.

WO 89/11850 discloses amphipathic liposomes for drug delivery consisting of an amphipathic bilayer-forming material and a passenger molecule entrapped in the bilayer itself (cf. page 3, lines 1-9). Liposomes comprising retinoic acid as a passenger molecule are described in Example 63 thereof. However, the bilayer-forming material of the liposomes of Example 63 consists of soy lecithin only.

In accordance with the present invention, a retinoid composition is provided as is defined in the claims. Furthermore, a pharmaceutical unit dosage formulation of a retinoid composition as defined in the claims is also provided. In still another aspect of the present invention, said retinoid composition or said formulation for use in a therapeutic method of inhibiting the growth of cancer cells as defined in the claims is provided. In addition, the use of said composition or said formulation for the manufacture of a medicament for the treatment of cancer as defined in the claims is provided. Further embodiments of the invention are also defined in the claims.

Therapeutically useful, reduced toxicity compositions of carotenoids can be provided. The compositions comprise a carotenoid, lipid carrier particles, and an intercalation promoter agent. "Carotenoid" is used here to include retinoids, proretinoids, carotenes, xanthophylls, and analogs thereof. A preferred example is all-trans retinoic acid. The carotenoid is substantially uniformly distributed with the lipid in the lipid carrier particles. More particularly, the carotenoid is substantially uniformly distributed in an intercalated position throughout a hydrophobic portion of the lipid carrier particles, as opposed to the aqueous phase. "Substantially uniformly distributed" means that at least 50% of the lipid carrier particles will contain carotenoid in a molar ratio between about 5:85 carotenoid:lipid and about 15:70. Preferably at least 75% of all lipid carrier particles will contain such a ratio of the active ingredient.

The composition is stable in an aqueous environment. In this context, "stable in an aqueous environment" means that the composition (1) will not exhibit any therapeutically significant degradation over a period of at least 24 hours, (2) will not exhibit a substantial degree of fusions of liposomes over that same period, and (3) will not exhibit substantial redistribution of the carotenoid over that same period, including no substantial movement of the drug into the aqueous phase of a liposome, and no substantial state change into a crystalline form.

The molar ratio of carotenoid to lipid in the lipid carrier particles is greater than about 1:10, and is most preferably at least about 15:85. The intercalation promoter agent preferably comprises at least about 15% by weight of the composition, and can suitably be, for example, a triglyceride.

"Lipid carrier particles" is used here to include liposomes, having a bilayer structure formed of one or more lipids having polar heads and nonpolar tails, as well as micelles, amorphous particulates of lipid, and other lipid emulsion state entities. When the particles are liposomes, suitable forms include multilamellar liposomes.

A pharmaceutical unit dosage formulation of a carotenoid, which comprises a carotenoid, lipid carrier particles, an intercalation promoter agent, and a pharmaceutically acceptable carrier can also be provided. As stated above, the carotenoid is substantially uniformly distributed with the lipid in the lipid carrier particles, and the composition is stable in an aqueous environment.

A method of inhibiting the growth of cancer cells, in which a therapeutically effective amount of a carotenoid composition is administered to a living subject can also be provided. The carotenoid composition can be as described above. The composition is preferably administered to the subject in a maintained molar ratio between about 5:85 carotenoid:lipid and about 15:70. "Maintained" in this context means that the stated ratio of drug to lipid lasts for at least 24 hours.

The present invention provides the therapeutic benefits of the carotenoid all-trans retinoic acid (tretinoin), while substantially reducing the undesirable toxicity of the composition, as compared to the free drug. The encapsulation of retinoic acid in liposomes results in a decrease of at least 15-fold in toxicity as compared to the free drug.

Further, the presence of the intercalation promoter agent permits the ratio of active ingredient to lipid to be increased above what has been previously known, and thus makes such formulations useful in a practical sense for lyophilization into a powder, and subsequent reconstitution into solution which can be administered parenterally to a patient. Without wishing to be bound by any particular theory, it is believed that the intercalation promoter agent overcomes steric hindrance that otherwise limits the amount of carotenoid that be incorporated in, for example, a liposome.

The encapsulation of carotenoids within, e.g., liposomes, permits their direct delivery to intracellular sites and thus circumvents the requirement for cell surface receptors. This may be of particular significance, for example, in therapy of tumors which lack the cell surface receptors for serum retinol binding protein but possess intracellular receptors for retinoic acid.

The Composition of the present invention is also substantially improved over prior liposomal retinoid formulations in terms of uniformity of drug distribution. Prior compositions often had substantial percentages of liposomes which contained essentially no drug. In the present invention, at least 50% and preferably at least 75% of all liposomes in the composition contain drug with the range specified above.

Figure 1 shows a time profile of liposomal retinoic acid (L-RA) stability in the presence (•) and absence (○) of serum.

Figure 2 shows human red blood cell (RBC) lysis as a function of time with RA (•) and L-RA (▲).

Figure 3 shows RBC lysis as a function of retinoic acid (RA) concentration (•) and L-RA concentration (▲).

Figure 4 shows the inhibition of THP-1 cell growth as a function of RA concentration (•), L-RA concentration (○) or empty liposome concentration (△).

Figure 5 shows the induction of transglutaminase (TGase) in human monocytic THP-1 cells as a function of treatment with RA or L-RA.

Figure 6 shows the inhibition of human histiocytic U-937 cell growth as a function of RA concentration (•), L-RA concentration (○) and empty liposome concentration (△).

Figure 7 shows the time course of accumulation of tissue TGase activity in cultured human peripheral blood monocytes (HPBM). HPBM were fractionated into small (○) and large (•) subpopulations by centrifugal elutriation, and they were cultured in 35-mm-well tissue culture plates as described in Materials and Methods. At the indicated time points the cells were washed, sonicated, and assayed for TGase activity. Values are the means of six determinations from two dishes.

Figure 8 shows dose-dependent effects of recombinant interferon-gamma (rIFN-g) on induction of tissue TGase activity in HPBM subpopulations. Small (○) and large (•) monocytes were cultured in serum containing medium alone or medium containing increasing concentrations of rIFN-g. After 72 hr, the cells were harvested and the cell lysates assayed for tissue TGase activity. The results shown represent mean ± SD of three determinations from an individual donor.

Figure 9 shows effects of retinol (ROH) and RA on induction of tissue TGase activity in cultured HPBM. Cells were cultured in the presence of 5% human AB serum and the absence (○) or presence of 500 nM ROH (▲) or RA (•) for varying periods of time. At the end of each time point, the cells were harvested and assayed for enzyme activity. Values shown are the means ± SD of six determinations from two independent experiments. Inset, dose-response curve for tissue TGase induction by ROH (▲) and RA (•) in HPBM after 72-hr culture.

Figure 10 shows effects of free- and liposome-encapsulated RA on induction of tissue TGase in HPBM. A: The cells were cultured in tissue culture dishes in presence of serum-containing medium alone (A) 500 nm liposomal RA (•), or medium containing 500 nM free-RA (▲), or "empty liposomes" (○) for indicated periods of time. Both the liposomal RA and "empty liposomes" contained 200 ug/ml lipid. At the end of each time point, the cultures were washed and cell lysates assayed for TGase activity. Values shown are the mean ± SD of six determinations from two independent experiments. B: Western-blot analysis of the levels of tissue TGase in freshly isolated HPBM (lane 1) and in HPBM cultured for 72 hr in the presence of serum-containing medium alone (lane 2), in medium containing 500 nM free RA (lane 3), 500 nM liposomal RA (lane 4), or "empty liposomes" (lane 5). Cell lysates containing 25 ug of protein were subjected to Western-blot analysis as described in Materials and Methods.

Figure 11 shows effect of free and liposome-encapsulated ROH on induction of tissue TGase in HPBM. A: HPBM monolayers were cultured in serum-containing medium alone (△) or medium containing 1 uM of free- (○) or liposomal-ROH (▲) for 72 hr. Then the cultures were washed and the cell lysates assayed for enzyme activity as described in Materials and Methods. B: Western-blot analysis of tissue TGase levels in freshly isolated HPBM (lane 1) and in HPBM cultured for 72 hr in the presence of serum-containing medium alone (lane 2), in medium containing 1 uM of free ROH (lane 3), or liposome-encapsulated ROH (lane 4) as described in Materials and Methods. Twenty-five micrograms of cell protein was loaded onto each lane.

Suitable therapeutic carotenoids for encapsulation include various retinoids. Trans-retinoic acid and all-trans-retinol are preferred. Other retinoids that are believed suitable include: retinoic acid methyl ester, retinoic acid ethyl ester, phenyl analog of retinoic acid, etretinate, retinol, retinyl acetate, retinaldehyde, all-trans-retinoic acid, and 13-cis-retinoic acid.

Lipid carrier particles, such as liposomes, can be formed by methods that are well known in this field. Suitable phospholipid compounds include phosphatidyl choline, phosphatidic acid, phosphatidyl serine, sphingolipids, sphingomyelin, cardiolipin, glycolipids, gangliosides, cerebrosides, phosphatides, sterols, and the like. More particularly, the phospholipids which can be used include dimyristoyl phosphatidyl choline, egg phosphatidyl choline, dilauryloyl phosphatidyl choline, dipalmitoyl phosphatidyl choline, distearoyl phosphatidyl choline, 1-myristoyl-2-palmitoyl phosphatidyl choline, 1-palmitoyl-2-myristoyl phosphatidyl choline, 1-palmitoyl-2-stearoyl phosphatidyl choline, 1-stearoyl-2-palmitoyl phosphatidyl choline, dioleoyl phosphatidyl choline, dimyristoyl phosphatidic acid, dipalmitoyl phosphatidic acid, dimyristoyl phosphatidyl ethanolamine, dipalmitoyl phosphatidyl ethanolamine, dimyristoyl phosphatidyl serine, dipalmitoyl phosphatidyl serine, brain phosphatidyl serine, brain sphingomyelin, dipalmitoyl sphingomyelin, and distearoyl sphingomyelin.

Phosphatidyl glycerol, more particularly dimyristoyl phosphatidyl glycerol (DMPG), is not preferred for use in the present invention. In the carotenoid compositions described herein, the presence of DMPG correlates with the appearance of amorphous structures of anomalous size, which are believed to render the composition much less suitable for intravenous administration. When DMPG is omitted, the amorphous structures are not observed. The undesirable effects that are apparently caused by the presence of DMPG may result from the fact that DMPG has a negative charge, which may interact with the carboxylate of the carotenoid.

In addition, other lipids such as steroids and cholesterol may be intermixed with the phospholipid components to confer certain desired and known properties on the resultant liposomes. Further, synthetic phospholipids containing either altered aliphatic portions, such as hydroxyl groups, branched carbon chains, cyclo derivatives, aromatic derivatives, ethers, amides, polyunsaturated derivatives, halogenated derivatives, or altered hydrophilic portions containing carbohydrate, glycol, phosphate, phosphonate, quaternary amine, sulfate, sulfonate, carboxy, amine, sulfhydryl, imidazole groups and combinations of such groups, can be either substituted or intermixed with the phospholipids, and others known to those skilled in the art.

A suitable intercalation promoter agent will permit the desired high molar ratio of carotenoid to lipid, without substantial crystallization from the liposomes after they are reconstituted in aqueous solution, as can be observed by microscopic analysis, separation techniques based on buoyant density, or other techniques well known to those skilled in the art. Triglycerides are preferred intercalation promoter agents, with soybean oil as one specific example. Other suitable agents include sterols, such as cholesterol, fatty alcohols, fatty acids, fatty acids esterified to a number of moieties, such as polysorbate, propylene glycol, mono- and diglycerides, and polymers such as polyvinyl alcohols.

Prior to lyophilization, the carotenoid, lipids, and intercalation promoter agent can be dissolved in an organic solvent, such as t-butanol. Lyophilization to form a preliposomal powder can be performed using commercial apparatus which is known to persons skilled in this field. After lyophilization, the powder can be reconstituted as, e.g., liposomes, by adding a pharmaceutically acceptable carrier, such as sterile water, saline solution, or dextrose solution, with agitation, and optionally with the application of heat.

A preferred formulation, which can be dissolved in 45 ml of t-butanol, is as follows:

| component | mg | millimoles | mole % | wt % |
|---|---|---|---|---|
| DMPC | 850 | 1.28 | 72 | 77 |
| soybean oil | 150 | 0.17 | 9 | 14 |
| tretinoin | 100 | 0.33 | 19 | 9 |

The composition of the present invention is preferably administered to a patient parenterally, for example by intravenous, intraarterial, intramuscular, intralymphatic, intraperitoneal, subcutaneous, intrapleural, or intrathecal injection. Administration could also be by topical application or oral dosage. Preferred dosages are between 40-200 mg/m². The dosage is preferably repeated on a timed schedule until tumor regression or disappearance has been achieved, and may be in conjunction with other forms of tumor therapy such as surgery, radiation, or chemotherapy with other agents.

The present invention is useful in the treatment of cancer, including the following specific examples: hematologic malignancies such as leukemia and lymphoma, carcinomas such as breast, lung, and colon, and sarcomas such as Kaposi's sarcoma.

### COMPARATIVE EXAMPLE 1

### Preparation of liposomal-all trans-retinoic acid (L-RA)

Preparation of lyophilized powder containing all trans-retinoic acid and phospholipids was carried out as follows. A solution of retinoic acid in t-butanol (1-5 mg/ml) was added to a dry lipid film containing dimyristoyl phosphatidyl choline (DMPC) and dimyristoyl phosphatidyl glycerol (DMPG) at a 7:3 molar ratio. The phospholipids were solubilized in the t-butanol containing the all-trans retinoic acid and the solution was freeze-dried overnight. A powder containing dimyristoyl phosphatidyl choline (DMPC), dimyristoyl phosphatidyl glycerol (DMPG), and all-trans retinoic acid was obtained. The lipid:drug ratio used was from 10:1 to 15:1.

Reconstitution of liposomal retinoic acid from the lyophilized powder was done as follows. The lyophilized powder was mixed with normal saline at room temperature to form multilamellar liposomes containing all trans-retinoic acid. This reconstitution method required mild hand-shaking for 1 min to obtain a preparation devoid of any aggregates or clumps. By light microscopy, the reconstituted preparation contained multilamellar liposomes of a close size range. No aggregates or drug clumps were identified in the liposomal preparation in three different experiments.

Encapsulation efficiency and size distribution of the liposomal all-trans retinoic acid preparation were determined as follows. The liposomal all-trans retinoic acid preparation was centrifuged at 30,000 x g for 45 minutes. A yellowish pellet containing the retinoic acid and the lipids was obtained. By light microscopy, the pellet was composed of liposomes with no crystals or drug aggregates. The encapsulation efficiency was calculated to be greater than 90% by measuring the amount of free retinoic acid in the supernatant by UV spectrophotometry. Liposomes were sized in a Coulter-Counter and Channelizer. The size distribution was as follows: 27% of liposomes less than 2 micrometers (um), 65% between 2 um and 3 um, 14% between 3 um and 5 um, 1% more than 5 um. The method used for encapsulation of retinoids was simple, reproducible and could be used for large scale production, for example, for clinical trials.

Further experiments were performed by the same procedure but with different lipids, ratios of lipids and the use of ³H-all-trans retinoic acid. Additional lipids utilized were dipalmitoyl phosphatidyl choline (DPPC) stearylamine (SA) and cholesterol. After sedimentation of the liposomes, residual ³H was determined and encapsulation efficiency calculated. Table 1 shows encapsulation efficiencies determined by this method for various L-RA preparations.

**TABLE 1**

| Encapsulation Efficiency of Retinoic Acid in Liposomes | |
|---|---|
| LIPOSOME COMPOSITION | ENCAPSULATION EFFICIENCY (%) |
| DMPC:cholesterol 9:1 | 69.3 |
| DMPC:cholesterol 9:3 | 64.5 |
| DPPC | 69.1 |
| DMPC:SA:cholesterol 8:1:1 | 56.7 |
| DMPC:DMPG 7:3 | 90 |
| DMPC:DMPG 9:1 | 90.7 |

Of the lipid compositions studied, DMPC:DMPG at ratios between 7:3 and 9:1 gave superior encapsulation efficiencies. Liposomal all-trans retinol (L-ROH) was prepared by the methods described above for L-RA with DMPC:DMPG, 7:3.

### COMPARATIVE EXAMPLE 2

### Stability of Liposomal Retinoic Acid

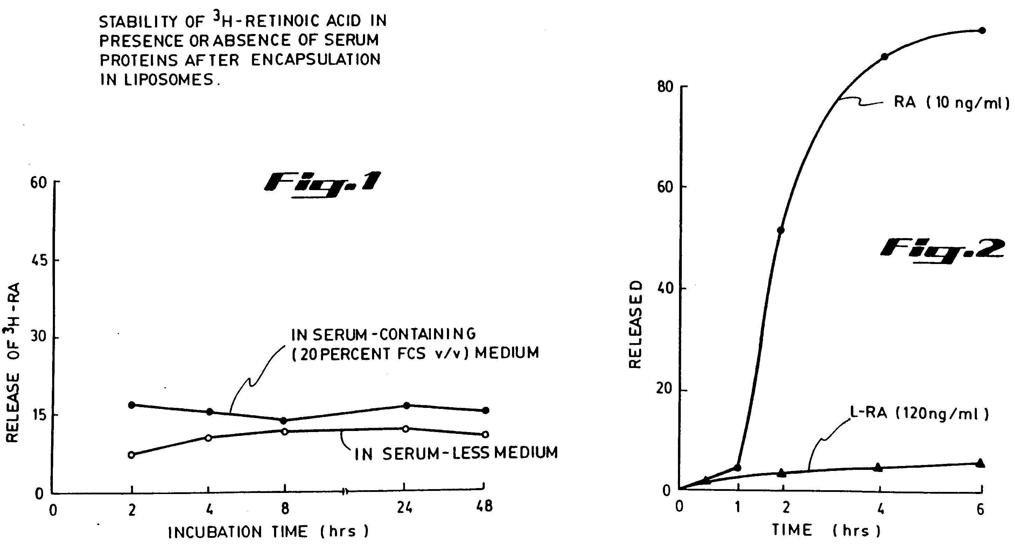

Liposomal ³H-retinoic acid (L-³H-RA) was prepared with DMPC:DMPG, 7:3 as described in Example 1. Samples of the L-³H-RA were incubated with either phosphate-buffered saline (PBS) or PBS with 20% (by volume) fetal calf serum (FCS). After various periods of incubation at about 37°C, aliquots were removed and centrifuged to sediment liposomes. The tritium in the supernatant solution was measured to determine ³H-RA release. Figure 1 shows the release of ³H-RA over a two day period. The L-³H-RA was over about 80% stable over the period of the experiment, even in the presence of 20% FCS.

When ³H-all-trans retinol was used to label L-ROH and stability in PBS measured, only about 5% of the ³H-ROH was released after a 24 hr incubation at 37°C.

### COMPARATIVE EXAMPLE 3

### In Vitro Lysis of Human Erythrocytes (RBCs) by Retinoic Acid or Liposomal Retinoic Acid

Lysis of human red blood cells (RBCs) was quantitated by measuring the release of hemoglobin in the supernatants by observation of increases in optical density at 550 nanometers (nm), as described previously (Mehta, et al., Biochem. Biophys, Acta., Vol. 770-, pp 230-234 (1984). Free-RA dissolved in dimethyl formamide (DMFA), was added to the RBCs. Results with appropriate solvent controls, empty liposomes, and empty liposomes plus free-drug were also noted. Release of hemoglobin by hypotonic lysis of the same number of human RBCs by water was taken as a 100% positive control, while cells treated with PBS were taken as negative controls.

Preparations of L-RA comprising various lipids were incubated at a concentration of 20 microgram (µg) RA per ml with RBCs in PBS for 4 hr at 37°C. The toxicity of the L-RA preparations on the basis of percent RBC lysis is shown in Table 2.

**TABLE 2**

| In Vitro Toxicity Of L-RA Preparations To RBCs | |
|---|---|
| LIPOSOME COMPOSITION | % RBC LYSIS |
| DMPC:Cholesterol | 4.5 |
| 9:1 | |
| DMPC:Cholesterol | 90.2 |
| 9:3 | |
| DPPC | 6.7 |
| DMPC:SA:Cholesterol | 70.4 |
| 8:1:1 | |
| DMPC:DMPG | 8 |
| 7:3 | |
| DMPC:DMPG | 8.3 |
| 9:1 | |

As may be seen from the data of Table 2, L-RA of DMPC:cholesterol, DPPC, DMPC:DMPG (7:3) and DMPC:DMPG (9:1) exhibited low RBC toxicity under these conditions. It is of interest to note that the latter two L-RA compositions exhibited superior encapsulation efficiencies (Table 1).

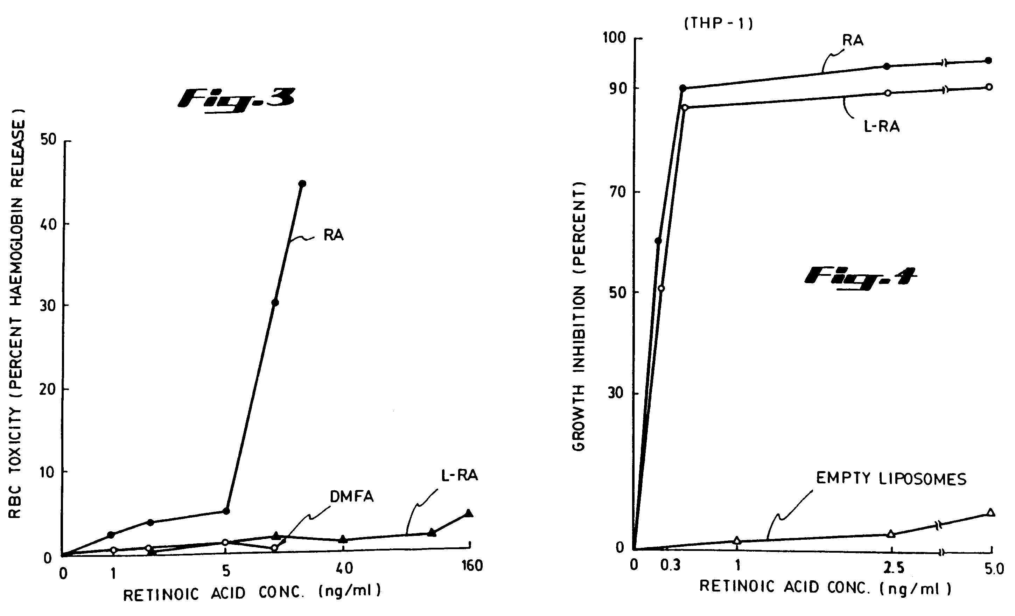

A further experiment concerning the toxicity over time of free RA and L-RA (DMPC:DMPG-7:3) toward RBC was conducted. Human erythrocytes were incubated at 37°C in PBS with 10 ug/ml free RA or 120 ug/ml L-RA, and RBC lysis monitored over a period of 5 hr. Figure 2 shows time courses of RBC lysis. At between about 1 hr and about 3 hr, the free RA extensively lysed a large majority of the erythrocytes. When a similar manipulation was performed with L-RA (DMPC:DMPG(7:3)) at a RA concentration of 120 ug/ml, little RBC lysis occurred (e.g., less than 10% after 6 hr).

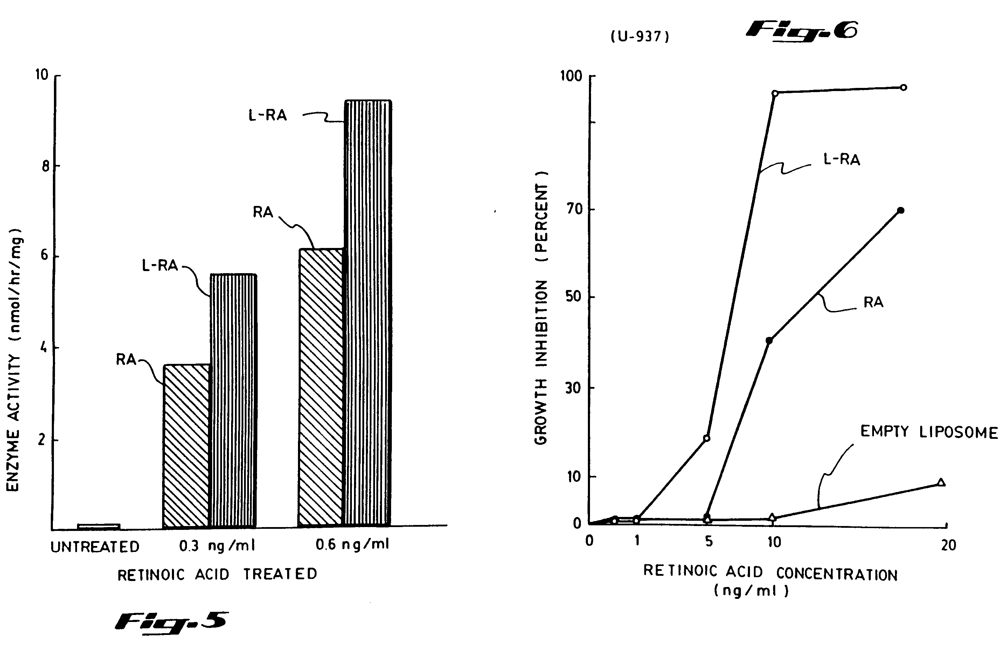

A study was also conducted concerning the effects upon RBC lysis in 2 hr of free RA and L-RA (DMPC:DMPG(7:3)) at various concentrations. Figure 3 shows the results of this study. Free RA showed linearly increasing RBC lysis between about 5 ug RA/ml and about 30 ug RA/ml. Liposomal RA caused RBC lysis of only about 5% at a concentration of 160 ug RA/ml.

### COMPARATIVE EXAMPLE 4

### Acute Toxicity Of Free And Liposomal Retinoic Acid

The acute toxicity of free and liposomal all-trans retinoic acid was studied in CD1 mice. Free all-trans retinoic acid was prepared as an emulsion in normal saline containing 10% DMSO and 2% Tween 80 at a concentration of 3 to 5 mg/ml. Liposomal all-trans retinoic acid was prepared using a lipid:drug ratio of 15:1. The final concentration of all-trans retinoic acid in the liposomal preparation was 3 mg/ml. Empty liposomes of the same lipid composition (DMPC:DMPG 7:3) were also tested at doses equivalent to 80 mg/kg, 100 mg/kg, and 120 mg/kg of liposomal-all trans retinoic acid. Normal saline containing 10% DMSO and 2% Tween 80 was also tested as a control at a dose equivalent to 50 mg/kg of free all-trans retinoic acid. All drugs tested were injected intravenously via tail vein as a single bolus. The injected volumes of free and liposomal-all-trans retinoic acid were the same for each dose.

Table 3 shows data obtained from these acute toxicity experiments.

**TABLE 3**

| Acute Toxicity of Free and Liposomal All-Trans Retinoic Acid | | | |
|---|---|---|---|
| Drug | Dose (mg/kg) | Number Animals with seizures | Number Animals alive (72 hr) |
| Free RA | 10 | 0/6 | 6/6 |
| | 20 | 6/6 | 5/6 |
| | 30 | 6/6 | 4/6 |
| | 40 | 3/3 | 0/3 |
| | 50 | 3/3 | 0/3 |
| L-RA | 40 | 0/6 | 6/6 |
| | 60 | 0/6 | 6/6 |
| | 80 | 0/6 | 6/6 |
| | 100 | 0/6 | 6/6 |
| | 120 | 0/6 | 6/6 |
| Empty Liposomes | 80 | 0/6 | 6/6 |
| | 100 | 0/6 | 5/6 |
| | 120 | 0/6 | 6/6 |
| Normal saline 10% DMSO 2% Tween 80 | 50 | 0/6 | 6/6 |

The maximum non-toxic dose of free all-trans retinoic acid was 10 mg/kg. Higher doses caused seizures immediately after injection. The acute LD₅₀ (deaths occurring up to 72 hours after injection) of free all-trans retinoic acid was 32 mg/kg. The cause of death was cardiopulmonary arrest after seizures for 1-2 minutes in all animals. No seizures or deaths were observed in the animals treated with liposomal all-trans retinoic acid at a dose of 120 mg/kg (maximum non-toxic dose and LD₅₀ greater than 120 mg/kg). Higher doses were not tested. No seizures were observed in the animals treated with empty liposomes or normal saline with 10% DMSO and 2% Tween 80.

### COMPARATIVE EXAMPLE 5

### In Vitro Inhibition of Tumor Cell Growth

Liposomal all-trans retinoic acid (L-RA) was prepared as described in Example 1.

Cells of the human monocytic cell line THP-1 were inoculated into samples of eucaryotic cell culture medium in the presence or absence of L-RA, at a final RA concentration of 1 micromolar (um). After 24 hr at 37°C, ³H-thymidine was added to each culture and incorporation thereof into cellular polynucleotides measured. Table 4 shows the percentage of tumor growth inhibition as reflected by decreases in ³H-thymidine incorporation induced by L-RA of differing lipid compositions.

**TABLE 4**

| L-RA Inhibition of Tumor Cell Growth | |
|---|---|
| LIPOSOME COMPOSITION | TUMOR CELL (THP-1) INHIBITION (%) |
| DMPC:Cholesterol | 72 |
| 9:1 | |
| DMPC:Cholesterol | 22 |
| 9:3 | |
| DPPC | 8 |
| DMPC:SA:Cholesterol | 84 |
| 8:1:1 | |
| DMPC:DMPG | 70 |
| 7:3 | |
| DMPC:DMPG | 32 |
| 9:1 | |

From Table 4, it should be noted that L-RA (DMPC:DMPG-7:3), which, as previously shown herein, gave a superior encapsulation efficiency and showed a low RBC toxicity (Tables 1 and 2), also effectively inhibited the tumor cell growth.

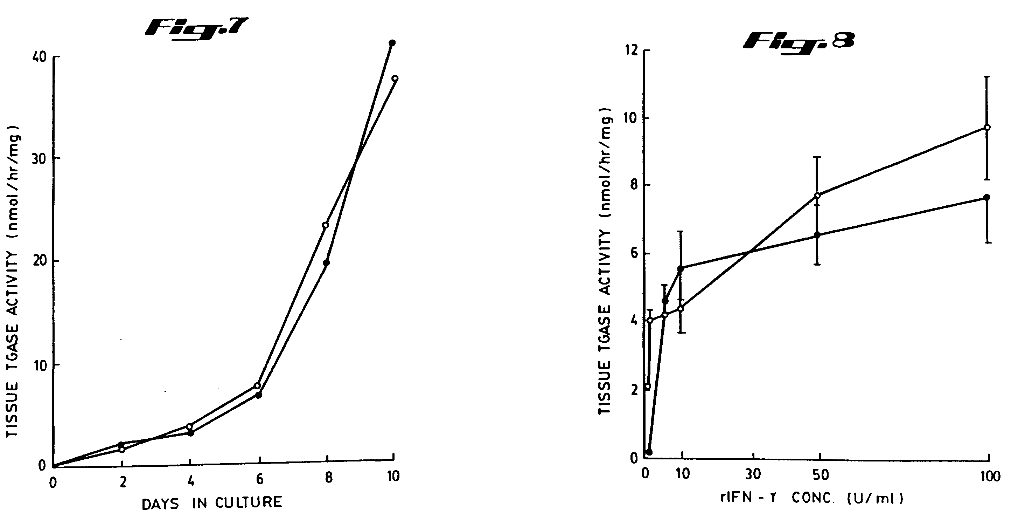

Cells of the human monocytic cell line THP-1 and of the human histiocytic cell line U-937 were inoculated at about 20,000 cells per well in aliquots of eucaryotic cell culture medium contained in wells of a 96 well microtiter plate. The medium in various wells contained different amount of free RA or L-RA (DMPC:DMPG 7:3). The cells were incubated for 72 hr at 37°C and cell growth determined and compared to that of controls without any form of retinoic acid. Figure 4 shows the inhibition of THP-1 cell growth by increasing concentrations of free RA or L-RA (DMPC:DMPG 7:3). At concentrations of less than 1 µg RA/ml, both preparations inhibited cell growth by over 90%.

The human monocytic leukemia THP-1 cells, after a 72 hr incubation with either free RA or L-RA at a concentration of 0.3 µg RA/ml, were observed to have lost their generally ovate form and to have a more flattened and spread morphological appearance often associated with cellular differentiation. The generally ovate form was retained when the cells were cultured in the absence of any free or liposomal retinoic acid.

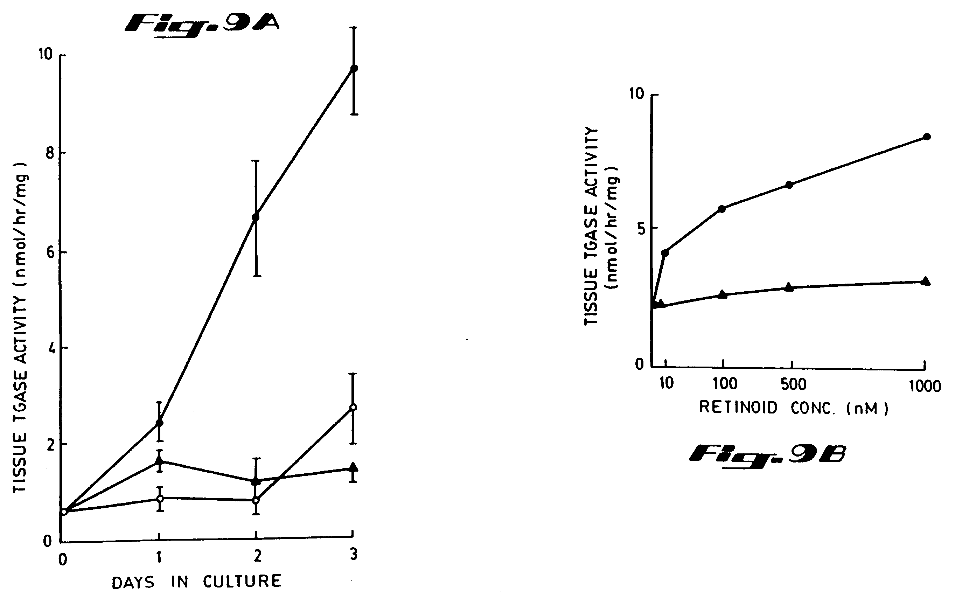

After incubation for 24 hr with 0.3 µg/ml or 0.6 µg/ml RA or L-RA in another experiment, THP-1 cells had increased levels of tissue transglutaminase enzymic activity, a marker for monocytic cell differentiation. As shown in Figure 5, THP-1 cells, at 4 x 10⁶ cells/ml, showed about 50% greater transglutaminase activity when incubated with L-RA as compared to free RA at equivalent retinoic acid concentrations.

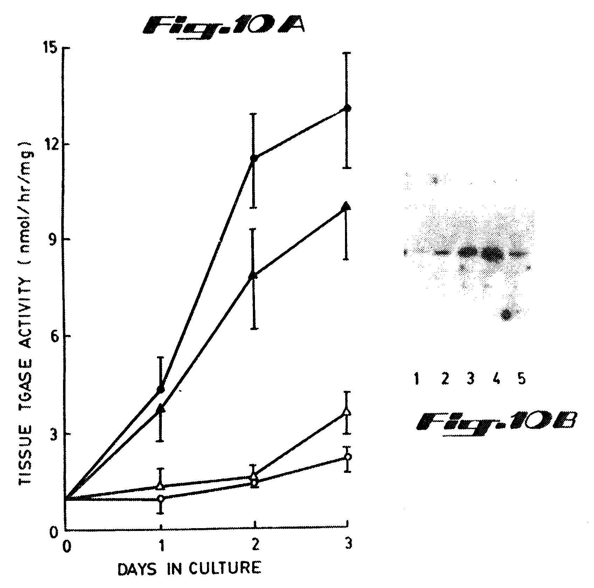

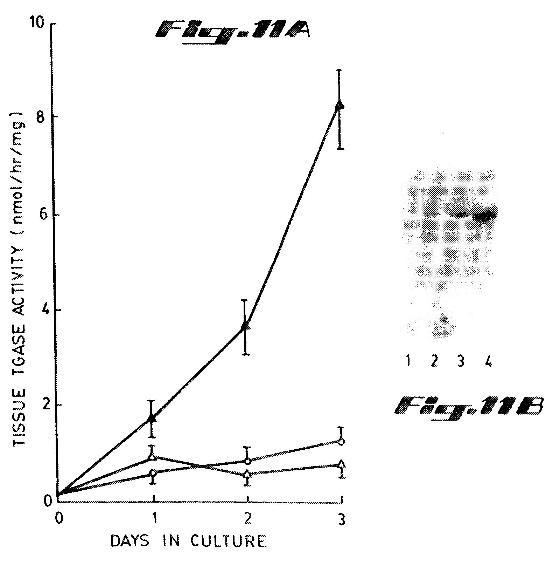

Cells of the human histiocytic cell line U-937 were distributed and cultured under the same conditions as the THP-1 cells in the prior experiment. Figure 6 shows the effects upon cell growth of increasing concentrations of free all-trans retinoic acid (RA), liposomal (DMPC:DMPG 7:3) all-trans retinoic acid (L-RA) and empty liposomes (which were devoid of retinoic acid). It should be noted that the U-937 cells were almost completely growth-inhibited by L-RA at a retinoic acid concentration of about 10 ug/ml while this amount of free RA inhibited growth less than 50%.

### COMPARATIVE EXAMPLE 6

### Antitumor Activity of Liposomal All-Trans Retinoic Acid in vivo

The antitumor activity of liposomal-all trans retinoic acid (DMPC:DMPG 7:3) was tested in vivo against liver metastases of M5076 reticulosarcoma. C57BL/6 mice were inoculated with 20,000 M5076 cells on day 0. Intravenous treatment with 60 mg/kg liposomal all-trans retinoic acid was given on day 4. The mean survival of control animals (non-treated) was 21.8 ± 1.6 days. The mean survival of treated animals was 27.0 ± 1.6 days. Liposomal all-trans retinoic acid was shown, therefore, to have antitumor activity at a dose well below the maximum non-toxic dose, against a cell line (M5076) which was resistant to free retinoic acid in in vitro studies. THP-1 cells treated in vitro with RA (1 MM) for 72 hours when injected subcutaneously into male mice, failed to develop into tumors, whereas untreated cells formed a huge mass of tumors in such mice.

### D. REFERENCES

1. Bankhurst, A.D., Hastein, E., Goodwin, J.S., and Peake, J.E. The nature of the prostaglandin-producing mononuclear cells in human peripheral blood. J. Lab. Clin. Med. 97, 179, 1981.
2. Baum, M., and Fisher, S. Macrophage production by bone marrow of tumor bearing mice. Cancer Res. 32, 2813, 1972.
3. Cuatrecasas, P. Hormone receptors, membrane phospholipids and protein kinases. Harvey Lect. 80, 89, 1986.
4. Davies, P.J.A., Murtaugh, M.P., Moore, W.T., Johnson, G.S., and Lucas, D.S. Retinoic acid-induced expression of tissue transglutaminase in human promyelocytic leukemia (HL-60) cells, J. Biol. Chem. 260, 5116, 1985.
5. Dennert, G. Retinoids and the immune system; Immunostimulation by vitamin A. In the Retinoids, Vol. 2 (Sporn, M.B., Roberts, A.B., and Goodman, D.S., Eds.) New York: Academic Press, p. 373, 1984.
6. Dizon, Q.S., and Southam, C.M. Abnormal cellular response to skin abrasions in cancer patients. Cancer 16, 1288, 1963.
7. Ferrero, D., Pressano, S., Pagliardi, G.L., and Robera. G. Induction of differentiation of human myeloid leukemias: surface changes probed with monoclonal antibodies. Blood 61, 171, 1983.
8. Ganguly, J., Roa, M.R.S., Murthy, S.K., and Sarada, K. Systemic mode of action of vitamin A. Vitam. Horm. 38, 1, 1980.
9. Heller J. Interaction of plasma retinol-binding protein with its receptors: specific binding of bovine and human retinol-binding proteins to pigment epithelium cells from bovine eyes. J. Biol. Chem. 248, 3613, 4975.
10. Leu, R.W., Herriot, M.J., Moore, P.E., Orr, G.R., and Birckbichler, P.J. Enhanced transglutaminase activity associated with macrophage activation. Exp. Cell Res. 141, 191, 1982.
11. Liebovich, S.J., and Ross, R. The role of the macrophage in wound repair. A study with hydrocortisone and antimacrophage serum. Am. J. Pathol. 78, 71, 1975.
12. Lopez-Berestein, G., Reuben, J., Hersh, E.M., Kilbourn, R., Hester, J.P., Bielski, M., Talpaz, M., and Mavligit, G.M. Comparative functional analysis of lymphocytes and monocytes from plateletapheresis. Transfusion 23, 201, 1983.
13. Lorand, L., Rule, N.G., Ong, H.G., Furlanetto, R., Jacobsen, A., Downey, J., Over, N., and Lorand, J. Amine specificity in transpeptidation. Inhibition of fibrin cross-linking. Biochemistry 7, 1214, 1980.
14. Lowry, O.H., Rosebrough, N.J., Farr, A.L., and Randall, R.J. Protein measurement with folin-phenol reagent. J. Biol. Chem. 193, 265, 1951.
15. Mehta, K., Claringbold, P., and Lopez-Berestein, G. Amphotericin B. inhibits the serum-induced expression of tissue transglutaminase in murine peritoneal macrophages. J. Immunol. 136, 4306, 1986.
16. Mehta, K., Juliano, R.L., and Lopez-Berestein, G. Stimulation of macrophage protease secretion via liposomal delivery of muramyl dipeptide derivatives to intracellular sites. Immunology 51, 517, 1984.
17. Mehta, K., and Lopez-Berestein, G. Expression of tissue transglutaminase in cultured monocytic leukemia (THP-1) cells during differentiation. Cancer Res. 46, 1388, 1986.
18. Mehta, K. Lopez-Berestein, G., Hersh, E.M., and Juliano, R.L. Uptake of liposomes and liposome-encapsulated muramyl dipeptide by human peripheral blood monocytes. J. Reticuloendothelial Soc. 32, 155, 1982.
19. Mehta, K., Lopez-Berestein, G., Moore, W.T., and Davies, P.J.A. Interferon-g requires serum retinoids to promote the expression of tissue transglutaminase in cultured human blood monocytes. J. Immunol. 134, 2053, 1985.
20. Mehta, K., Murtaugh, M.P., Juliano, R.L., and Davies, P.J.A. Phagocytosis inhibits the expression of tissue transglutaminase in mouse peritoneal macrophages J. Immunol. 132, 2552, 1984.
21. Moore, W.T., Murtaugh, M.P., and Davies, P.J.A. Retinoic acid induced expression of tissue transglutaminase in mouse peritoneal macrophages. J. Biol. Chem. 259, 12794, 1984.
22. Murtaugh, M.P., Arend, W.P., and Davies, P.J.A. Induction of tissue transglutaminase in human peripheral blood monocytes. J. Exp. Med. 159, 114, 1984.
23. Murtaugh, M.P., Dennison, O., Stein, J.P., and Davies, P.J.A. Retinoic acid induced gene expression in normal acid leukemic myeloid cells. J. Exp. Med. 163, 1325, 1986.
24. Murtaugh, M.P., Mehta, K., Johnson, J., Meyers, M., Juliano, R.I., and Davies, P.J.A. Induction of tissue transglutaminase in mouse peritoneal macrophages. J. Biol. Chem. 258, 11074, 1983.
25. Nelson, D.S. Macrophages as effectors of cell-mediated immunity. CRC Crit. Rev. Microbiol 1, 45, 1972.
26. Normann, S.J., and Weiner, R. Cytotoxicity of human peripheral blood monocytes. Cell. Immunol. 81, 413, 1983.
27. Norris, D.A., Morris R.M., Sanderson, R.J., and Kohler, P.F. Isolation of functional subsets of human peripheral blood monocytes. J. Immunol. 123, 166, 1979.
28. Olsson, I.L., and Brietman, T.R. Induction of differentiation of the human histiocytic lymphoma cell line U-937 by retinoic acid and cyclic adenosine 3', 5'-monophosphate inducing agents. Cancer Res. 42, 3924, 1982.
29. Peterson, P.A. Characteristics of a vitamin A transporting protein complex occurring in human serum. J. Biol. Chem. 246, 34, 1971.
30. Picker, L.J., Raff, H.V., Goldyne, M.E., and Stobo, J.B. Metabolic Heterogeneity among human monocytes and its modulation of PGE₂. J. Immunol. 124, 2557, 1980.
31. Rask, L., and Peterson, P.A. In vitro uptake of vitamin A from the retinol-binding plasma protein to mucosal epithelial cells from the monkey's small intestine. J. Biol. Chem. 251, 6360, 1976.
32. Roberts, A.B., and Sporn, M.B. Cellular biology and biochemistry of the retinoids, In The Retinoids, Vol. 2 (Sporn, M.B., Roberts A.B., and Goodman, D.S., Eds.) New York: Academic Press, p. 209, 1984.
33. Rothblat, G.H., Arborgast, L.Y., Quellett, L., and Howard, B.V. Preparation of delipidized serum proteins for use in cell culture systems. In Vitro 12, 554, 1976.
34. Schroff, G., Neumann, C., and Sorg, C. Transglutaminase as a marker for subsets of murine macrophages. Eur. J. Immuno. 11, 637, 1981.
35. Scott, K. F., Meyskens, F.L., Jr., and Russel, D.H. Retinoids increase transglutaminase activity and inhibit ornithine decarboxylase activity in Chinese ovary hamster cells and in melanoma cells stimulate to differentiate. Proc. Natl. Acad. Sci. USA, 79, 4053, 1982.
36. Turpin, J., Hester, J.P., Hersh, E.M., and Lopez-Berestein. G. Centrifugal elutriation as a method for isolation of large number of functionally intact human peripheral blood monocytes. J. Clin. Apheresis. 3, 111, 1986.
37. Turpin, J., Hersh, E.M., and Lopez-Berestein, G. Characterization of small and large human peripheral blood monocytes; effects of in vitro maturation on hydrogen peroxide release and on the response to macrophage activators. J. Immunol. 136, 4194, 1986.
38. Yuspa, S. Ben, T., and Litchi, U. Regulation of epidermal transglutaminase activity and terminal differentiation by retinoids and phorbol esters. Cancer Res. 43, 5707, 1983.
39. Yuspa, S., Ben, T., and Steinert, P. Retinoic acid induces transglutaminase activity but inhibits cornification of cultured epidermal cells. J. Biol. Chem. 257, 9906, 1982.

## Claims

1. A retinoid composition, comprising about 77 % by weight of dimyristoyl phosphatidyl choline (DMPC), about 14 % by weight of soybean oil, and about 9 % by weight of all-*trans* retinoic acid (tretinoin).

2. A pharmaceutical unit dosage formulation of a retinoid composition, comprising the composition of claim 1 and a pharmaceutically acceptable carrier.

3. The formulation of claim 2, wherein the tretinoin is intercalated in liposomes.

4. The formulation of claim 2 or 3, wherein the ratio of lipid in the liposomes to the total liquid volume of the formulation is no greater than about 1g/50ml.

5. The formulation of any of claims 2 to 4, wherein the formulation contains at least about 100mg of tretinoin.

6. The formulation of any of claims 2 to 5, where the total liquid volume of the formulation is no greater than about 50ml.

7. The composition of claim 1 or the formulation of any of claims 2 to 6 for use in a therapeutic method of inhibiting the growth of cancer cells.

8. The use of the composition of claim 1 or the formulation of any of claims 2 to 6 for the manufacture of a medicament for the treatment of cancer.

## Patentansprüche

1. Retinoidzusammensetzung, umfassend etwa 77 Gew.-% Dimyristoylphosphatidylcholin (DMPC) etwa 14 Gew.-% Sojaöl und etwa 9 Gew.-% alltrans-Retinoesäure (Tretinoin) .

2. Pharmazeutisches Einheitsdosierungspräparat einer Retinoidzusammensetzung, umfassend die Zusammensetzung nach Anspruch 1 und einen pharmazeutisch verträglichen Träger.

3. Präparat nach Anspruch 2, wobei das Tretinoin in Liposomen interkaliert ist.

4. Präparat nach Anspruch 2 oder 3, wobei das Verhältnis von Lipid in den Liposomen zum gesamten Flüssigkeitsvolumen des Präparats nicht mehr als etwa 1 g/50 ml beträgt.

5. Präparat nach einem der Ansprüche 2 bis 4, wobei das Präparat mindestens 100 mg Tretinoin enthält.

6. Präparat nach einem der Ansprüche 2 bis 5, wobei das gesamte Flüssigkeitsvolumen des Präparats nicht mehr als etwa 50 ml beträgt.

7. Zusammensetzung nach Anspruch 1 oder Präparat nach einem der Ansprüche 2 bis 6 zur Verwendung in einem therapeutischen Verfahren zur Hemmung des Wachstums von Krebszellen.

8. Verwendung der Zusammensetzung nach Anspruch 1 oder des Präparats nach einem der Ansprüche 2 bis 6 zur Herstellung eines Arzneimittels zur Behandlung von Krebs.

## Revendications

1. Une composition rétinoïde, comprenant environ 77 % en poids de dimyristoyl phosphatidyl choline (DMPC), environ 14 % en poids d'huile de soja et environ 9 % en poids d'acide rétinoïde tout-*trans* (trétinoïne).

2. Une formulation pharmaceutique en dose unitaire d'une composition rétinoïde, comprenant la composition de la revendication 1 et un véhicule pharmaceutiquement acceptable.

3. La formulation de la revendication 2, dans laquelle la trétinoïde est intercalée dans des liposomes.

4. La formulation de la revendication 2 ou 3, dans laquelle le rapport des lipides dans les liposomes par rapport au volume liquide total de la formulation n'est pas supérieur à environ 1 g/50 ml.

5. La formulation de l'une des revendications 2 ou 4, dans laquelle la formulation contient au moins environ 100 mg de trétinoïne.

6. La formulation de l'une des revendications 2 ou 5, où le volume liquide total de la formulation n'est pas supérieur à environ 50 ml.

7. La formulation de la revendication 1 ou la formulation de l'une des revendications 2 à 6, pour utilisation dans un procédé thérapeutique d'inhibition de la croissance de cellules cancéreuses.

8. L'utilisation de la composition de la revendication 1 ou de la formulation de l'une des revendications 2 à 6, pour la fabrication d'un médicament pour le traitement du cancer.
